# EUROPEAN PATENT SPECIFICATION

(11) **EP 2 956 193 B1**
(45) Date of publication and mention of the grant of the patent: **19.07.2017**
(21) Application number: 14714341.6
(22) Date of filing: 13.02.2014
(51) Int. Cl.: A61M 5/42, A61B 17/30, A61B 17/34

(54) **TOOL FOR THE INDUCTION OF THE PNEUMOPERITONEUM AND ASSEMBLY COMPRISING THE TOOL**
INSTRUMENT ZUR INDUKTION DES PNEUMOPERITONEUMS UND ANORDNUNG MIT DEM INSTRUMENT
OUTIL PERMETTANT L'INDUCTION DU PNEUMOPÉRITOINE ET ENSEMBLE COMPRENANT L'OUTIL

(30) Priority: 13.02.2013 IT EN20130002
(43) Date of publication of application: 23.12.2015
(73) Proprietor: Gicastart S.r.l., 90128 Palermo (IT)
(72) Inventor: Cassata, Giovanni, 90145 Palermo (IT)
(74) Representative: Maiello, Helenio Francesco
(86) International application number: PCT/IB2014/058974
(87) International publication number: WO 2014/125434

(56) References cited:
- EP-A1- 1 779 788
- WO-A1-97/26828
- WO-A1-2009/015030
- WO-A2-2007/047813
- GB-A- 2 489 492
- US-A- 5 882 344

## Description

### Technical Field

The present invention relates to the surgical instruments and in particular has as object a tool adapted to be used during induction of the pneumoperitoneum, generally during laparoscopy.

The invention also relates with an assembly comprising the above tool and a laparoscopy needle, adapted to be used in combination with each other.

### State of the art

The induction of the pneumoperitoneum is a technique used in surgery, particularly in laparoscopic surgery, whose aim is to insufflate a gas inside the abdominal cavity, usually carbon dioxide, to distance the abdominal wall from the internal organs and create in this way a "work chamber", i.e. a zone having a volume suitable to allow the insertion of surgical tools.

One of the possible way of induction of pneumoperitoneum provides the use of a special needle with a variable length, called Veress needle, adapted to be introduced into the abdominal cavity realizing a small incision on the skin after having induced the pneumoperitoneum.

In most cases, the lifting of the abdominal wall is performed by grasping the same with both hands, being thus particularly inconvenient and difficult to perform.

Therefore, the rate of operations carried out using the Veress needle or similar tools is rather low, in particular close to 20%.

From the state of the art some accessories are known whose aim is to facilitate the lifting of the abdominal wall to make it easier and safer for the subsequent step of insertion of the needle.

For example CN201542695 discloses a pincer type tool whose object is to allow the gripping of the abdomen and the lifting of the abdominal wall.

However, this tool, in addition to provide an uncomfortable handling, is absolutely not safe and can easily cause damage and injury to the abdominal tissues.

Other solutions, disclosed for example in WO2011/128713, US2008/0058851, US2004/0049127, provide the use of a suction device having a bowl-shaped element substantially hemispherical or cap-shaped adapted to be placed in contact with its open edge on the skin of the patient.

The bowl-shaped element has a first center-offset opening and connected with vacuum means adapted to create a vacuum thereinside to promote the adhesion of the skin with the internal wall of the bowl-shaped element and a second opening having an axis coincident with the central axis of the bowl-shaped element and designed to allow the introduction of the needle.

A tool as disclosed above and provided with a rigid bowl-shaped element is also disclosed in WO2009/015030. Said document discloses features falling under the preamble of claim 1. The invention is defined in claim 1. Further embodiments of the invention are defined in the dependent claims. A first drawback of these solutions is represented by the presence of the inlet ducts connected to the first opening of the bowl-shaped element, which can represent an obstacle for the operator.

Moreover, the need to have a system for aspirating air, in addition to the need of providing the connection of the inlet with the bowl-shaped element at the beginning of each operation, makes these devices not of immediate use, as well as particularly expensive.

As a consequence such devices can be used only in premises that are suitably equipped and provided with suitable suction means.

A further drawback is represented by the fact that the introduction of the needle must be carried out with the same always in axial position, in practice perfectly orthogonal to the abdominal wall, thus requiring a not natural movement of the arm of the operator. US2010210915 also shows a suction device of the type disclosed above but with a bowl-shaped element having a decentralized opening for the insertion of a surgical tool.

In this case, however, in addition to the above drawbacks relating the aspiration, the introduction of the tool is not guided and therefore it requires a particular manual skill by the operator in order to not injure the internal organs.

Therefore, these drawbacks are not useful to contribute to the perception of the operation of induction of pneumoperitoneum as a safe procedure, thus not helping its diffusion.

Not least, in the tool according to the state of the art, the presence of a rigid structure, with an integral bowl-shaped element which has possibly a gripping and manipulation rod formed integrally thereto, does not allow to modify the configuration of the tool in order to adapt to the operative conditions preferred by the operator in turn using it or to change it in the light of further operational needs.

WO2007047813 discloses a device for lifting the abdomen in order to insert a needle, wherein the tool is a flexible bowl-shaped element designed to be compressed in order to provide the vacuum needed for lifting the abdominal wall. The tool is devoid of any element adapted to handle the flexible bowl-shaped element and to promote its deformation and to guide the insertion of the needle.

### Scope of the invention

An object of the present invention is to overcome the above drawbacks, realizing a tool for the induction of pneumoperitoneum which is simple and quick to use and which is also cost-effective.

A particular object is to provide a tool for the induction of pneumoperitoneum adapted to be used in combination with a needle for laparoscopy, such as a Veress needle or the like, which tool being easy to be handled and at the same time allowing the needle to be handled in a natural and safe way.

Still another object is to provide a tool for the induction of pneumoperitoneum which has low encumbrance.

A further object of the invention is to provide a tool for the induction of pneumoperitoneum which may be used in any kind of premise, without the need of auxiliary equipments.

Still another object is to provide a tool for the induction of pneumoperitoneum which is particularly safe for the patient undergoing the operation, be it human or animal.

A further object is to provide a tool for the induction of pneumoperitoneum which is customizable in its configuration to suit the operational needs of the patient and/or the operator carrying out the operation.

Not last object of the present invention is to provide a tool for the induction of pneumoperitoneum allowing the stroke of the needle to be adjusted to control the depth of penetration into the peritoneum.

These objects, as well as others which become more apparent hereinafter, are provided by a tool for inducing pneumoperitoneum which, according to claim 1, comprises a bowl-shaped element having an open end edge designed to come into contact relationship with the skin of a patient at the abdomen wall thereof, said bowl-shaped element having an outer surface, an inner surface designed to come at least partially into contact with the abdominal wall of a patient and at least one through-hole with an inner diameter at least equal to the outer diameter of the needle used in combination, for the passage thereof.

The bowl-shaped element is made of an elastically yieldable material to elastically deform and change, upon a pressure exerted by an operator on said outer surface, from an undeformed condition wherein said inner surface is spaced apart from the skin to a deformed condition wherein said inner surface is close to the abdominal wall to promote an at least partial vacuum upon its elastic return into said undeformed condition to lift the abdominal wall.

Thanks to this combination of features, the tool may be quickly and easily used without it being necessary to provide special auxiliary equipments, such as air intake means.

Moreover, the elasticity of the bowl-shaped element will allow the same to adapt to the anatomy of the peritoneum during its deformation, subsequently creating a depression area which will promote the not traumatic lift of the tissue, ensuring high safety for the whole operation.

Suitably, the bowl-shaped element may have the form of substantially hemispherical or spherical cap with a central axis of symmetry and the hole may have a central axis substantially parallel to the symmetry axis or inclined with respect to the same with an angle of inclination less than 45° and preferably between 1° and 5°.

The latter configuration will have the advantage of allowing the introduction of the needle into the peritoneum in a slightly inclined position, making easy the natural positioning of the arm by the operator.

Advantageously, the tool may comprise a substantially cylindrical guide rod having a free proximal end and the opposite distal end connected to said bowl-shaped element at said hole, said rod having an axial passage substantially coaxial with said hole for the guided insertion of the needle.

With this further feature, the needle will be guided during all the insertion step through the hole, providing a greater sense of safety.

In the text, the words "proximal" and "distal" are referred to the position of the portion to which they relates with respect of the operator handling the tool and the assembly.

Therefore, the word "proximal" relates to the portion closer to the operator and thus far from the abdomen of the patient, while the word "distal" relates to the opposite portion.

Further, the rod may have adjustable end-stroke means adapted to define an adjustable abutment for the needle for varying the stroke thereof.

By this way the tool may be always used in association with a needle of the same kind, regardless from the total length of the rod, but allowing the depth of insertion inside the peritoneum to be controlled, without it be necessary to provide a series of needle different for their useful lengths.

In a first configuration, the outer surface of said bowl-shaped element may have an annular projection arranged peripherally to said hole and having an inner peripheral wall with an inner diameter substantially corresponding to the outer diameter of said substantially cylindrical rod, said connecting means being of the screw and nut type and being associated with said inner wall of said projection and to the outer surface of said rod at said distal connection end.

According to an alternative embodiment, the guide rod may instead include or be constituted by a female tubular element and a male tubular element coupled to one another via their insertion into said hole of said bowl-shaped element from opposite sides with respect to the same.

Suitably, said male and female elements may present respective countershaped means for their mutual coupling, such as screw and nut means complementary with each other for the mutual coupling by screwing, each of said elements having a radial flange adapted to abut on axially opposite faces of a same circular portion of said bowl-shaped element peripheral with respect to said central hole, for tightening said rod thereon.

In both embodiments it will be possible to remove the rod if necessary, for example for sanitizing it separately from the bowl-shaped element, or to replace it with a rod having different size so as to adapt its configuration to the specific needs of the operator and/or patient.

Suitably, said male tubular element may be externally threaded, while said central hole of said bowl-shaped element may have a peripheral wall counter-threaded to allow the anchoring by screwing of said male element even in the absence of said tubular female element, so as to allow also the screwing of the female element to be adjusted and to vary the total height of the rod, adapting it to the length of the needle and/or the depth of penetration thereof selected each time by the operator.

Suitably, a plurality of guide rods may be arranged which are removably and selectively anchorable to said bowl-shaped element at said central hole and which are differentiated in size with each other.

In one of those variants, said rod of said plurality may comprise respective male tubular elements and female tubular elements adapted to be mutually coupled, said male elements, respectively female, being mutually differentiated by the length and/or the outer and/or inner diameter.

Suitably, the male and/or female elements may be provided with corresponding radial circular flanges for tightening said rod on said bowl-shaped element which flanges having a maximum outer diameter differentiated with respect to the flanges of the other male and/or female elements to vary the degree of deformability of said bowl-shaped element.

According to a further aspect of the invention an assembly for the induction of pneumoperitoneum is provided, which comprises, according to claim 15, an internally hollow needle having a substantially cylindrical elongated body with a gripping proximal end adapted to be handled by an operator and a pointed or sharpened opposite distal end adapted to penetrate into the abdominal cavity of a patient through the abdominal wall and a tool according to claim 1 adapted to be placed in contact with the patient's skin at its abdominal wall to lift the same and promote the insertion of said needle into the abdominal cavity.

Advantageous forms of the invention are achieved according with the dependent claims.

### Brief disclosure of the drawings

Further features and advantages of the invention will become more apparent in the light of a detailed description of two preferred but not limiting embodiments of a tool for induction of the pneumoperitoneum according to the invention, shown as not limiting examples with the aid of the appended drawings wherein:
FIG. 1 is a front view of the tool of the invention in a first preferred embodiment associated with a Veress needle;
FIG. 2 is a cross section view of the tool of Fig. 1;
FIG. 3 is an upper view of the tool of Fig. 1;
FIG. 4 is an exploded front view of a particular of the tool of Fig. 1;
FIG. 5 is a cross section view of the particular of Fig. 4 in assembled condition;
FIG. 6 is a front view of the tool of the invention in a second preferred embodiment associated with a Veress needle;
FIG. 7 is an upper view of the assembly of Fig. 6;
FIG. 8 is a cross section front view of the assembly of Fig. 6;
FIG. 9 is an exploded front view of the assembly of Fig. 6;
FIG. 10 is a cross section front view of the assembly of the invention in a third preferred embodiment.

### Best mode of carrying out the invention

With reference to the above figures, an assembly used in laparoscopic surgery for the induction of pneumoperitoneum is shown.

In its most general embodiment the assembly, generally indicated by 1, comprises a laparoscopic needle 2, of the Veress type or the like, adapted to be used in combination with a tool according to the present invention adapted to allow the lifting of the abdominal wall of a patient to promote the operation of insertion of the needle 2 and the subsequent induction of pneumoperitoneum according to traditional techniques.

The needle 2 may be any laparoscopic needle commercially available and generally it will comprise an elongated body 3 substantially cylindrical and internally hollow with a predetermined maximum outer diameter dₑ.

The elongated body 3 will have a gripping proximal end 4 provided with a shaped handle 5 adapted to be handled by an operator and also having a connector 6 to connect the tubular cavity 7 to a source of CO₂ or other fluid to be injected into the peritoneum, not shown since of the known type.

The opposite distal end 8 of the elongated body 3, which is also open, will be appropriately pointed or sharpened to penetrate into the abdominal cavity of the patient through the abdominal wall.

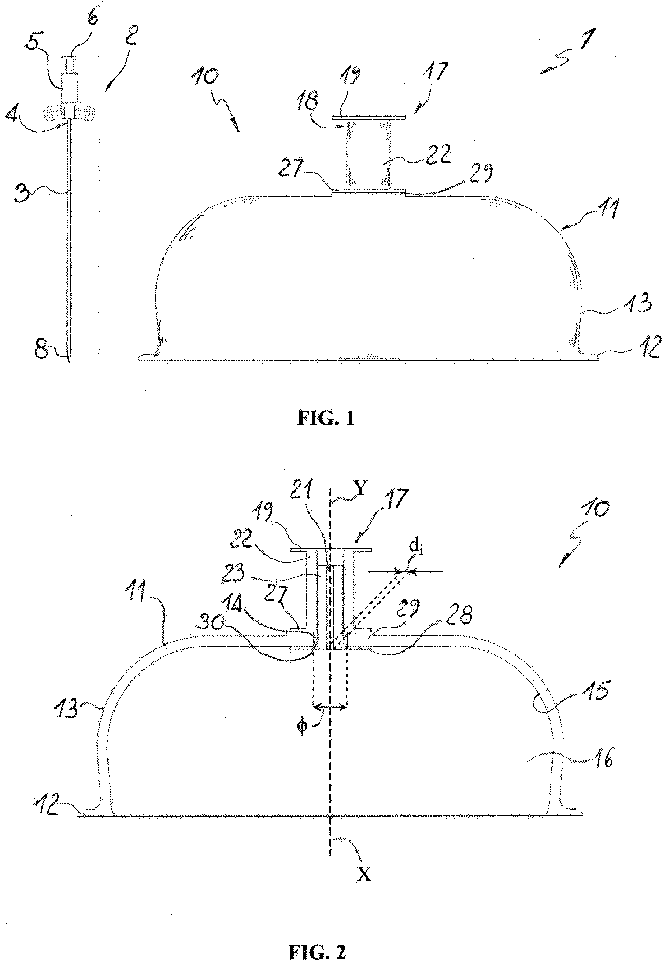

Fig. 1 shows a first preferred but not exclusive embodiment of a tool according to the invention, generally designated by 10, which essentially comprises a bowl-shaped element 11 having an open end edge 12, substantially circular or shaped as a closed loop, designed to come into contact with the skin of a patient at the abdominal wall to be lifted.

The bowl-shaped element 11 has a substantially hemispherical or spherical cap shape with an outer surface 13 defining a central axis of symmetry X and which is provided with at least one hole 14 with an inner diameter φ at least equal to the outer diameter dₑ of the needle 2 to be use in combination, for the passage thereof towards the skin.

The bowl-shaped element 11 is made of an elastically yielding material to elastically deform and move, in response to a pressure exerted by an operator on its outer surface 13 from the undeformed condition shown in Fig. 1, wherein it has an inner surface 15 spaced from the skin to a deformed position wherein the inner surface 15 is brought into close proximity or in partial adhesion to the skin.

In this way, the bowl-shaped element 11 will act as a suction cup, following the expulsion of air through the hole 14 and/or the edge 12, and at the time of its elastic return in the undeformed position will produce an at least partial vacuum in the chamber 16 between the abdominal wall and the inner surface 15, promoting the lifting of the abdominal wall and allowing the needle 2 to penetrate the same through the hole 14 without risk of damaging the internal organs.

The material used for the bowl-shaped element 11 may be rubber or other polymeric material with sufficient elastic yielding and porosity sufficient to adhere with the skin at its open edge 12, so as to create a chamber 16 wherein an at least partial vacuum is present. Preferably, the material will be biologically compatible and can be hot and/or cold sterilized.

Moreover, the material may be either opaque or transparent or translucent to allow the viewing of the abdominal wall during the entire operation.

In this first embodiment, shown in Figs. 1 to 5, and more clearly visible from the section of Fig. 2, the hole 14 may have a central axis Y substantially coincident with the central axis of symmetry X.

Furthermore, the hole 14 has a diameter φ substantially greater than the outer diameter dₑ of the needles typically used for such operations in order to allow the coupling of the bowl-shaped element 11 with a guide rod 17 whose function will also be that of allowing the manipulation of the bowl-shaped element 11 and the exertion of the pressure required for the deformation thereof.

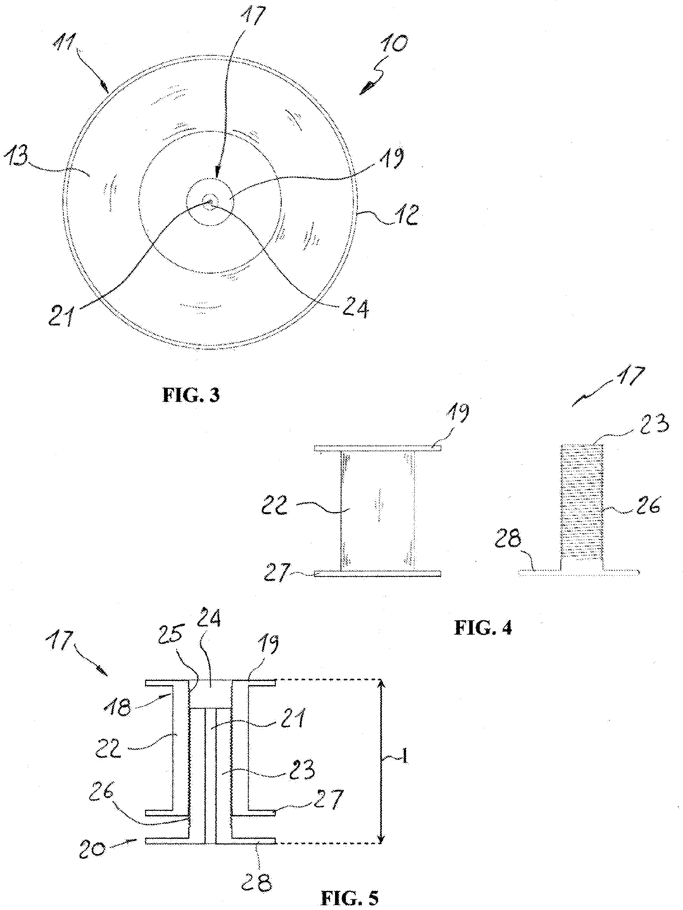

The guide rod 17 is substantially cylindrical with a free proximal end 18, provided with a grip ring 19, more clearly shown in Fig. 3, adapted to make safer the grip of the rod 17 by the operator, and the distal end 20 connected to the bowl-shaped element 11 in correspondence of the hole 14.

The rod 17 is crossed for all its length by an axial passage 21 open at both ends and substantially coaxial with hole 14, for the guided insertion of the needle 2.

Fig. 4 shows the particular configuration of the rod 17, formed by a female tubular element 22 and a male tubular element 23 adapted to be coupled with each other through their insertion into the hole 14 from opposite sides with respect thereto.

As shown in Fig. 5, the female element 22 and male element 23 are removably couplable with each other by means of respective complementary shaped screw and nut means associated with each other for the mutual screwing.

In particular, the female element 22 has a through cavity 24, with an inner diameter dᵢ preferably not larger than the diameter φ of the hole 14 and even more preferably slightly lower than the same, whose inner peripheral surface 25 is threaded.

The male element 23, in turn, is provided with the above passage 21 for the needle 2 and has an outer peripheral surface 26 counter-threaded with respect to the inner surface 25 of the female element 22.

Moreover, the female and male elements 22, 22 have each a radial flange 23, respectively 27 and 28, adapted to abut on axially opposite faces of a same circular portion 29 of the bowl-shaped element 11 peripheral to the central hole 14, for tightening the rod 17 thereon.

According to the shown embodiment, the central hole 14 has a substantially smooth peripheral wall 30 and the anchoring of the rod 17 will be obtained through the mutual tightening of the two flanges 27, 28 on the circular portion 29.

According to a not shown variant, the peripheral wall 30 of the hole may be conter-threaded with respect to the outer peripheral surface 26 of the male element 23 to allow the removable anchoring thereof by screwing to the bowl-shaped element 11 even in the absence of the tubular female element 22.

This latter may be subsequently screwed to the male element 23 with adjustable screw depth to adjust the overall length 1 of the rod 17.

Alternatively, the same male element 23 may be associated with different female elements 22 differentiated with each other in the axial length and/or in its outer diameter.

In both the two variants of the first shown embodiment disclosed above, it will be possible to provide a series of guide rods 17 adapted to be selectively and removably anchored to the bowl-shaped element in correspondence of the central hole 14.

The rods 17 of the series may be differentiated with each other for one or more dimensions (length, inner diameter, outer diameter) of the respective tubular female elements 22 and male elements 23.

According to a particular variant, the female 22 and/or male elements 23 of the rods 17 of the series may be differentiated with respect to the female 22 and/or male elements 23 of the other rods 17 in the outer diameter of the respective circular tightening flange 27,28.

In this way it will be possible to vary the deformability degree of the bowl-shaped element as a function of the vacuum degree to be obtained and consequently of the lifting degree of the abdominal wall.

As matter of fact, this parameter is also selected according to the depth of penetration of the needle 2 in the abdominal wall, also determined by the operator based on anatomical characteristics of the patient.

In particular, in the case where it is necessary to penetrate the abdominal wall in a limited way, it is preferable to keep the same more spaced apart from the inner surface 15 of the bowl-shaped element.

Therefore it is necessary to produce a lower vacuum degree and to this end it is possible to use a female element 22 and/or male element 23 having a flange 27, 28 of small diameter, so as to produce a smaller deformation of the bowl-shaped element 11 and a lower vacuum degree.

In contrast, the flanges 27, 28 of larger diameter will allow to obtain the most greater deformation of the bowl-shaped element 11 and therefore also a higher degree of vacuum.

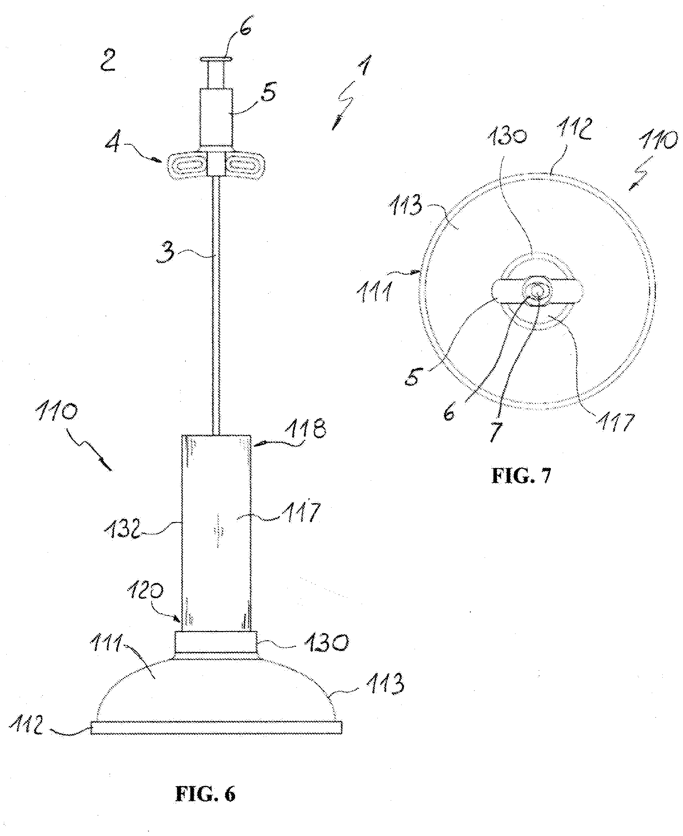

Fig. 6 shows a second embodiment of a tool according to the invention, generally designated by 110, having a bowl-shaped element 111 with a deformable outer surface 113 shaped as a spherical cap, an open peripheral edge 112 and an inner surface 113 designed to be brought in proximity to the abdominal wall, possibly in at least partial contact therewith, at the time of deformation so as to induce an at least partial vacuum in the inner chamber 116 in response to the elastic return in the undeformed position.

The bowl-shaped element 111 has a central hole 114 with a central axis Y substantially parallel to the central axis of symmetry X of the outer surface 113.

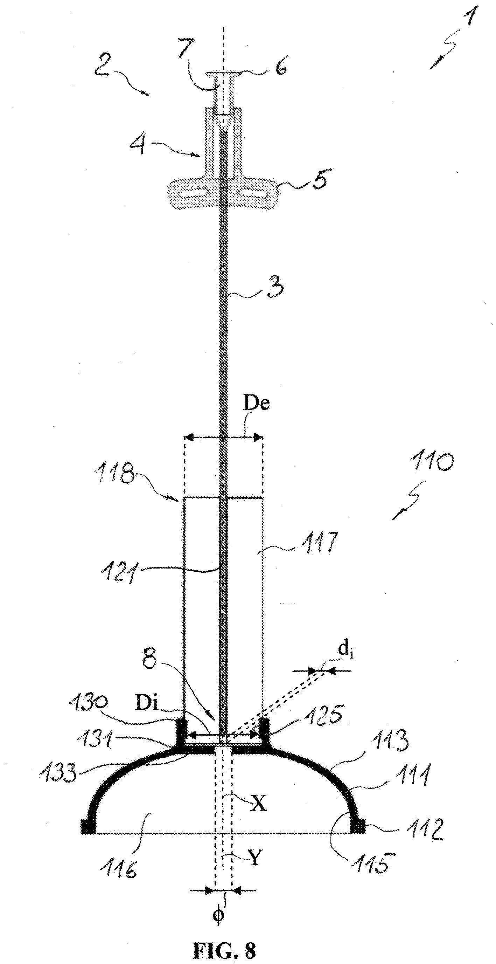

Although, as shown in Fig. 8, the central hole 114 may have an inner diameter φ substantially corresponding to the outer diameter dₑ of the cylindrical body 3 of the needle 2, due to the elasticity of the bowl-shaped element 111, in the undeformed position the hole 114 may also have an inner diameter φ less than the outer diameter dₑ of the needle 2.

As matter of fact, in this case the pointed end 8 of the latter may still cause the enlargement, allowing the easy passage of the elongated body 3 toward the skin of the abdominal wall.

The bowl-shaped element 111 will also be fastened to a substantially cylindrical guide rod 117 having a free proximal end 118 and the distal end 120 connected to the bowl-shaped element 111 at the hole 114.

In particular, the rod 117 will be crossed for all its length by an axial passage 121 open at both ends and substantially coaxial with the hole 114, for the guided insertion of the needle 2.

The bowl-shaped element 111 differs from the bowl-shaped element 11 of the first embodiment above disclosed in particular for the provision of an annular projection 130 arranged peripherally to the hole 114 and having an inner peripheral wall 131 with a diameter Di substantially corresponding to the outer diameter De of the substantially cylindrical rod 117.

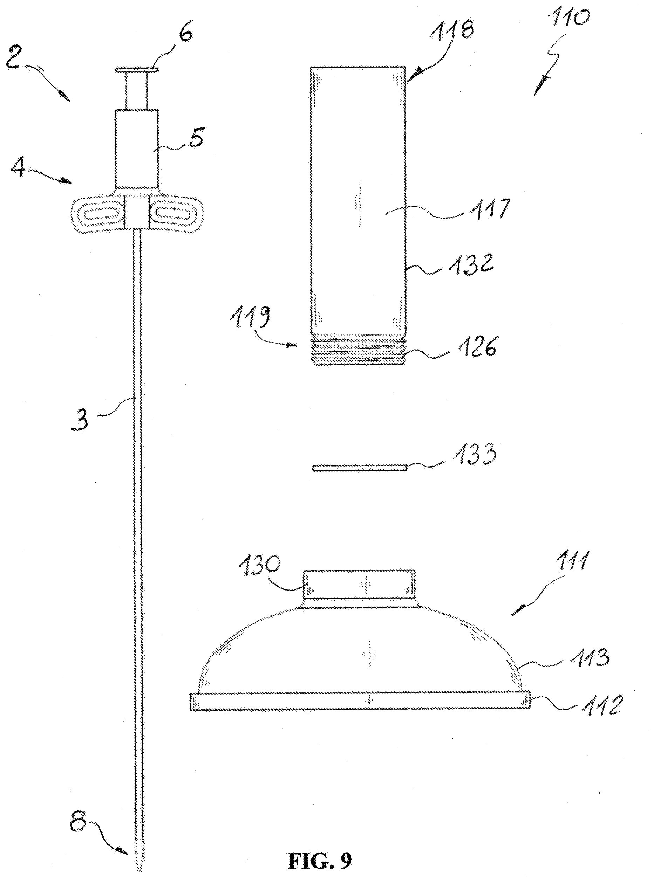

As shown in Fig. 9, the distal end 120 of the rod 117 is connected with the bowl-shaped element 111 by removable connection means of the screw and nut type, defined by a thread 125 formed on the inner wall 131 of the annular projection 130 and a counterthread 126 formed on the outer surface 132 of the rod 117, in correspondence of the same distal end 120.

Moreover a sealing washer 133 may be interposed between the latter and the outer surface 113 of the bowl-shaped element 111.

Analogously to the first embodiment shown in Fig. 1, the tool 110 may be provided with a series of rods 117 mutually differentiated in their lengths.

In a not shown variant, the bowl-shaped element 111 and the rod 117 may instead be integral with each other and not adapted to be uncoupled.

According to further variants of the two disclosed embodiments, also not shown, the rod 17, 117 may have adjustable end-stroke means adapted to define an adjustable abutment for the needle 2 so as to adjust the axial stroke and consequently the depth of penetration.

For example, the end-stroke means may include a slider adapted to axially slide on the rod 17, 117 or with respect thereto to interact with a part of the needle 2, for example with the handle 6 or with a counter abutment, not shown, associated with the elongated body 3, limiting or increasing the maximum stroke of the needle 2.

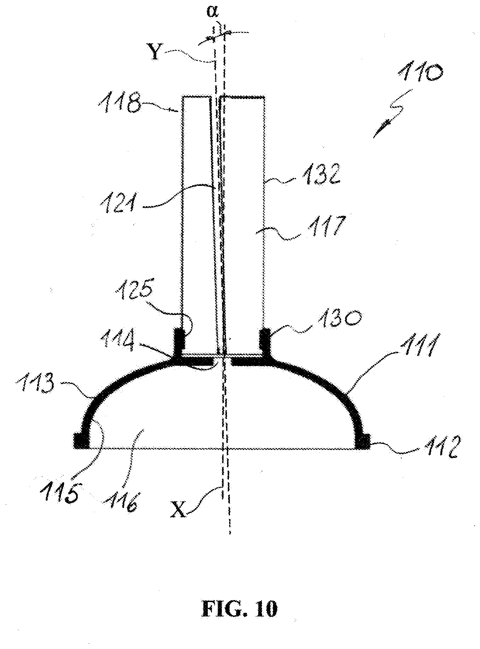

Fig. 10 shows a further variant of the tool 110 of the invention which differs from the embodiment of Fig. 6 essentially for the fact that the inner passage 121 of the rod 117 has an axis of development Z inclined with respect to the central axis of symmetry X with a predetermined inclination angle α.

Preferably, the angle of inclination α will be less than 45° and even more preferably between 1° and 5°.

In this case, the central hole 114 may have substantially elongated shape in plan and not more circular and therefore its central axis Y will also be inclined with the same angle of inclination α.

A further variant, not shown, the rod 17, 117 is provided with a transverse auxiliary channel in fluidic communication with the axial passage 21, 121 and adapted to allow the draining of air at the time of deformation of the bowl-shaped element 11, 111, in particular in the case in which the axial passage 21, 121 is clogged by the operator's hand.

From the foregoing it appears evident that the invention achieves the intended objects and in particular to provide a tool for the induction of pneumoperitoneum, which is of simple and immediate use, which is particularly safe and economical and which does not require special equipment auxiliary to be used.

The tool and the assembly according to the invention are susceptible of numerous modifications and variations, all falling within the inventive concept expressed in the appended claims. All the details may be replaced with other technically equivalent elements, and the materials may be different depending on requirements, without departing from the scope of protection of the present invention.

Although the tool and the assembly have been disclosed with particular reference to the appended drawings, reference numbers used in the description and in the claims are used to improve the intelligence of the invention and do not constitute any limitation to the claimed scope of protection.

## Claims

1. A tool for the induction of the pneumoperitoneum, adapted to be used in combination with a laparoscopy needle (2), such as a Veress needle or the like, having a substantially tubular elongated body (3) with a predetermined maximum outer diameter (dₑ), which tool (10, 110) comprises:
- a bowl-shaped element (11, 111) having an open end edge (12, 112) adapted to adhere with the skin of the patient at the abdominal wall, said bowl-shaped element (11, 111) having an outer surface (13, 113), an inner surface (15, 115) and at least one hole (14, 114) with an inner diameter (φ) at least equal to the outer diameter (d_{c}) of the needle (2) used in combination, for the passage thereof;
- a substantially cylindrical guide rod (17, 117) having a free proximal end (18, 118) and the opposite distal end (20, 120) fastened to said bowl-shaped element (11, 111) at said hole (14, 114), said rod (17, 117) having a passage (21, 121) substantially co-axial with said hole (14, 114) for the guided insertion of the needle (2);
wherein said bowl-shaped element (11, 111) is made of an elastically yieldable material to elastically deform and move, in response to a pressure exerted by an operator on said outer surface (13, 113), from an undeformed condition wherein said inner surface (15, 115) is spaced apart from the skin to a deformed condition wherein said inner surface (15, 115) is close to the abdominal wall or in at least partial contact therewith to produce an at least partial vacuum upon its elastic return in said undeformed condition, for lifting the abdominal wall;
**characterized in that** said guide rod (17) comprises a female tubular element (22) and a male tubular element (23) adapted to be removably coupled with each other by their insertion into said central hole (14) of said bowl-shaped element (11) from opposite sides with respect thereof, said female element (22) and said male element (23) of said guide rod (17) have corresponding nut and screw means (25, 26) complementary shaped with each other for the mutual coupling by screwing, each of said female and male elements (22, 23) having a radial flange (27, 28) adapted to abut against axially opposite faces of a same circular portion (29) of said bowl-shaped element (11, 111) arranged peripherally with respect of said central hole (14), for tightening said rod (17) thereon.

2. Tool as claimed in claim 1, **characterized in that** said bowl-shaped element (11, 111) is substantially hemisphere-shaped or spherical cap-shaped with a central symmetry axis (X), said hole (14) having a central axis (Y) substantially parallel to said central symmetry axis (X).

3. Tool as claimed in claim 1, **characterized in that** said bowl-shaped element (11, 111) is substantially hemisphere-shaped or spherical cap-shaped with a central symmetry axis (X), said hole (14, 114) having a central axis (Y) inclined with respect of said central symmetry axis (X) with an inclination angle (α) lower than 45° and preferably between 1° and 5°

4. Tool as claimed in claim 2 or 3, **characterized in that** said outer surface (113) of said bowl-shaped element (111) has an annular projection (130) arranged peripherally to said hole (114) and having an inner peripheral wall (131) with an inner diameter (Di) substantially equal to the outer diameter (De) of said substantially cylindrical rod (117), said connecting means being of the nut and screw type (125, 126) and being associated with said inner peripheral wall (131) of said projection (130) and to the outer surface (132) of said rod (117) at said connection distal end (120).

5. Tool as claimed in claim 4, **characterized in that** said tubular male element (23) is externally threaded, said central hole (14) of said bowl-shaped element (11) having a counter-threaded peripheral wall (30) to allow the anchoring by screwing of said male element (23) even in absence of said tubular female element (22).

6. Tool as claimed in claim 5, **characterized in that** said rod (17, 117) has an auxiliary transverse channel in fluidic communication with said axial passage (21, 122) and adapted to allow the drainage of air upon the deformation of said bowl-shaped element (11, 111).

7. Tool as claimed in claim 6, **characterized in that** said rod (17, 117) has adjustable end-stroke means adapted to define an adjustable abutment for the needle (2).

8. Tool as claimed in claim 7, **characterized by** comprising a plurality of guide rods (17, 117) adapted to be removably and selectively coupled with said bowl-shaped element (101, 111) at said central hole (14, 114).

9. Tool as claimed in claim 8, **characterized in that** said rods (17) of said plurality comprise respective male tubular elements (23) and tubular female elements (22) reciprocally coupable, said male elements (23), respectively female (22), being differentiated with each other in their lengths and/or outer and/or inner diameter.

10. Tool as claimed in claim 9, **characterized in that** said rods (17) of said plurality comprise respective pairs of tubular male elements (23) and tubular female elements (22) coupable with each other, the male elements (23) an/or female elements (22) of said pairs being differentiated with each other in the maximum outer diameter of the corresponding tightening flanges (27, 28) to adjust the degree of deformability of said bowl-shaped element (11).

11. Assembly for the induction of the pneumoperitoneunn, comprising:
- a needle (2) having a substantially tubular elongated body (3) with a proximal gripping end (4) adapted to be handled by an operator and an opposite sharpened distal end (8) adapted to penetrate into the abdominal cavity of a patient through the abdominal wall;
- a tool (10, 110) adapted to be placed into contact with the skin of a patient at its abdominal wall for lifting it and promote the introduction of said needle (2) in the abdominal cavity;
**characterized in that** said tool (10, 110) is according to one or more of the preceding claims.

## Patentansprüche

1. Werkzeug für die Induktion des Pneumoperitoneums, das angepasst ist, um in Kombination mit einer Laparoskopic-Nadel (2), verwendet zu werden, wie z. B. eine Veress-Nadel o. ä., mit einem im Wesentlichen röhrenförmigen gestreckten Körper (3) mit einem vorbestimmten maximalen Außendurchmesser (dₑ), wobei das Werkzeug (10, 110) folgendes umfasst:
- ein napfförmiges Element (11, 111) mit einem offenen Endrand (12, 112), der angepasst ist, um an der Haut des Patienten an der Bauchdccke anzuhaften, wobei das napfförmige Element (11, 111) eine Außenfläche (13, 113), eine Innenfläche (15, 115) und mindestens ein Loch (14, 114) mit einem Innendurchmesser (φ) hat, der mindestens gleich wie der Außendurchmesser (dₑ) der in Kombination verwendeten Nadel (2) für deren Durchgang ist;
- einen im Wesentlichen zylindrischen Führungsstab (17, 117) mit einem freien proximalen Ende (18, 118) und dem gegenüberliegenden distalen Ende (20, 120), das am napfförmigen Element (11, 111) an dem Loch (14, 114) befestigt ist, wobei der Stab (17, 117) einen Durchgang (21, 121) hat, der für die geführte Einführung der Nadel (2) im Wesentlichen koaxial mit dem Loch (14, 114) ist;
wobei das napfförmige Element (11, 111) aus einem elastisch nachgiebigen Material ist, um sich als Reaktion auf einen Druck, der von einem Bediener an der Außenfläche (13, 113) ausgeübt wird, von einem unverformten Zustand, wobei die Innenfläche (15, 115) von der Haut beabstandet ist, in einen verformten Zustand, wobei die Innenfläche (15, 115) nahe der Bauchdecke oder in mindestens teilweisem Kontakt damit ist, elastisch zu verformen und zu bewegen, um bei seiner elastischen Rückkehr in den unverformten Zustand zum Anheben der Bauchdecke ein mindestens teilweises Vakuum zu erzeugen; **dadurch gekennzeichnet, dass** der Führungsstab (17) ein röhrenförmiges Buchsenelement (22) und röhrenförmiges Stiftelement (23) umfasst, die angepasst sind, um entfernbar durch ihr Einführen in das mittlere Loch (14) des napfförmigen Elements (11) von gegenüberliegenden Seiten davon miteinander gekoppelt zu werden, wobei das Buchsenelement (22) und das Stiftelement (23) des Führungsstabs (17) entsprechende Mutter- und Schraubenmittel (25, 26) haben, die zum gegenseitigen Koppeln durch Schrauben komplementär zueinander geformt sind, wobei jedes der Buchsen- und Stiftelemente (22, 23) einen radialen Flansch (27, 28) hat, der angepasst ist, um an axial gegenüberliegenden Flächen eines gleichen kreisförmigen Abschnitts (29) des napfförmigen Elements (11, 111) anzuliegen, der peripher in Bezug auf das mittlere Loch (14) angeordnet ist, um den Stab (17) daran anzuziehen.

2. Werkzeug nach Anspruch 1, **dadurch gekennzeichnet, dass** das napfförmiges Element (11, 111) im Wesentlichen halbkugelförmig oder kugelhaubenförmig mit einer mittleren Symmetrieachse (X) ist, wobei das Loch (14) eine Mittelachse (Y) hat, die im Wesentlichen parallel zu der mittleren Symmetrieachse (X) ist.

3. Werkzeug nach Anspruch 1, **dadurch gekennzeichnet, dass** das napfförmiges Element (11, 111) im Wesentlichen halbkugelförmig oder kugelhaubenförmig mit einer mittleren Symmetricachse (X) ist, wobei das Loch (14, 114) eine Mittelachse (Y) hat, die in Bezug auf die mittlere Symmetrieachse (X) mit einem Neigungswinkel (α) von weniger als 45° und vorzugsweise zwischen 1° und 5° geneigt ist.

4. Werkzeug nach Anspruch 2 oder 3, **dadurch gekennzeichnet, dass** die Außenfläche (113) des napfförmigen Elements (111) einen ringförmigen Vorsprung (130) hat, der peripher zu dem Loch (114) angeordnet ist, und eine innere Umfangswand (131) mit einem Innendurchmesser (Di) hat, der im Wesentlichen gleich wie der Außendurchmesser (De) des im Wesentlichen zylindrischen Stabs (117) ist, wobei die Verbindungsmittel von der Art Mutter und Schraube (125, 126) sind und mit der inneren Umfangswand (131) des Vorsprungs (130) und mit der Außenfläche (132) des Stabs(117) an dem distalen Verbindungsende (120) assoziiert sind.

5. Werkzeug nach Anspruch 4, **dadurch gekennzeichnet, dass** das röhrenförmigen Stiftelement (23) ein Außengewinde hat, wobei das mittlere Loch (14) des napfförmigen Elements (11) eine Gegengewinde-Umfangswand (30) hat, um das Verankern durch Schrauben des Stiftelements (23) auch in Abwesenheit des röhrenförmigen Buchsenelements (22) zu ermöglichen.

6. Werkzeug nach Anspruch 5, **dadurch gekennzeichnet, dass** der Stab (17, 117) einen transversalen Hilfskanal in fluidischer Kommunikation mit dem axialen Durchgang (21, 122) hat, und der angepasst ist, um die Dränage von Luft bei der Verformung des napfförmigen Elements (11, 111) zu ermöglichen.

7. Werkzeug nach Anspruch 6, **dadurch gekennzeichnet, dass** der Stab (17, 117) ein einstellbares Endlagenmittel hat, das angepasst ist, um ein einstellbares Widerlager für die Nadel (2) zu definieren.

8. Werkzeug nach Anspruch 7, das **dadurch gekennzeichnet ist, dass** es eine Vielzahl von Führungsstäben (17, 117) umfasst, die angepasst sind, um entfernbar und selektiv mit dem napfförmigen Element (101, 111) an dem mittleren Loch (14, 114) gekoppelt zu werden.

9. Werkzeug nach Anspruch 8, **dadurch gekennzeichnet, dass** die Stäbe (17) der Vielzahl entsprechende röhrenförmige Stiftelemente (23) und röhrenförmigen Buchsenelemente (22) umfassen, die gegenseitig gekoppelt werden können, wobei sich die Stiftelemente (23) bzw. Buchsenelemente (22) in ihren Längen und/oder ihrem Außen- und/oder Innendurchmesser unterscheiden.

10. Werkzeug nach Anspruch 9, **dadurch gekennzeichnet, dass** die Stäbe (17) der Vielzahl jeweilige Paare von röhrenförmigen Stiftelementen (23) und röhrenförmigen Buchsenelementen (22) umfassen, die miteinander gekoppelt werden können, wobei sich die Stiftelemente (23) und/oder Buchsenelemente (22) der Paare untereinander im maximalen Außendurchmesser der entsprechenden Anzugsflansche (27, 28) zum Einstellen des Verformbarkeitsgrads des napfförmigen Elements (11) unterscheiden.

11. Einheit zur Induktion des Pneumoperitoneums, umfassend:
- eine Nadel (2) mit einem im Wesentlichen röhrenförmigen gestreckten Körper (3) mit einem proximalen Greifende (4), das angepasst ist, um von einem Bediener gehandhabt zu werden, und einem gegenüberliegenden geschärften distalen Ende (8), das angepasst ist, um in die Bauchhöhle eines Patienten durch die Bauchdecke zu penetrieren;
- ein Werkzeug (10, 110), das angepasst ist, um in Kontakt mit der Haut eines Patienten an seiner Bauchdecke gebracht zu werden, um diese anzuheben und das Einführen der Nadel (2) in die Bauchhöhle zu fördern;
**dadurch gekennzeichnet, dass** das Werkzeug (10, 110) nach einem oder mehreren der vorherigen Ansprüche ist.

## Revendications

1. Outil permettant l'induction du pneumopéritoine, adapté pour être utilisé en combinaison avec une aiguille de laparoscopie (2), telle qu'une aiguille Veress ou similaire, ayant un corps allongé de forme sensiblement tubulaire (3) avec un diamètre extérieur maximal prédéterminé (dₑ), lequel outil (10, 110) comprend :
- un élément en forme de bol (11, 111) ayant un bord d'extrémité ouvert (12, 112) adapté pour adhérer à la peau du patient au niveau de la paroi abdominale, ledit élément en forme de bol (11, 111) ayant une surface extérieure (13, 113), une surface intérieure (15, 115) et au moins un trou (14, 114) avec un diamètre intérieur (φ) au moins égal au diamètre extérieur (dₑ) de l'aiguille (2) utilisée en combinaison, pour le passage de celle-ci ;
- une tige de guidage sensiblement cylindrique (17, 117) ayant une extrémité proximale libre (18, 118) et l'extrémité distale opposée (20, 120) fixées audit élément en forme de bol (11, 111) au niveau dudit trou (14, 114), ladite tige (17, 117) ayant un passage (21, 121) sensiblement coaxial audit trou (14, 114) pour l'insertion guidée de l'aiguille (2); dans lequel ledit élément en forme de bol (11, 111) est constitué d'un matériau élastiquement déformable pour se déformer élastiquement et se déplacer, en réponse à une pression exercée par un opérateur sur ladite surface extérieure (13, 113), d'une condition non déformée dans laquelle ladite surface intérieure (15, 115) est écartée de la peau à une condition déformée dans laquelle ladite surface intérieure (15, 115) est proche de la paroi abdominale ou en contact au moins partiel avec celle-ci pour produire un vide au moins partiel lors de son retour élastique dans ladite condition non déformée, pour soulever la paroi abdominale ;
**caractérisé en ce que** ladite tige de guidage (17) comprend un élément tubulaire femelle (22) et un élément tubulaire mâle (23) adaptés pour être couplés l'un à l'autre par leur insertion dans ledit trou central (14) dudit élément en forme de bol (11) à partir de côtés opposés par rapport à celui-ci, ledit élément femelle (22) et ledit élément mâle (23) de ladite tige de guidage (17) ont des moyens correspondant à écrou et vis (25, 26) profilés de manière complémentaire l'un à l'autre pour l'accouplement mutuel par vissage, chacun desdits éléments femelle et mâle (22, 23) ayant une bride radiale (27, 28) adaptée pour venir en butée contre des faces axialement opposées d'une même portion circulaire (29) dudit élément en forme de bol (11, 111) agencée de manière périphérique par rapport audit trou central (14), pour serrer ladite tige (17) sur celui-ci.

2. Outil selon la revendication 1, **caractérisé en ce que** ledit élément en forme de bol (11, 111) est de forme sensiblement hémisphérique ou de forme de calotte sphérique avec un axe de symétrie central (X), ledit trou (14) ayant un axe central (Y) sensiblement parallèle audit axe de symétrie central (X).

3. Outil selon la revendication 1, caractérisé en cc que ledit élément en forme de bol (11, 111) est de forme sensiblement hémisphérique ou de forme de calotte sphérique avec un axe de symétrie central (X), ledit trou (14, 114) ayant un axe central (Y) incliné par rapport audit axe de symétrie central (X) avec un angle d'inclinaison (α) inférieur à 45° et de préférence entre 1° et 5°.

4. Outil selon la revendication 2 ou 3, **caractérisé en ce que** ladite surface extérieure (113) dudit élément en forme de bol (111) a une saillie annulaire (130) agencée de manière périphérique audit trou (114) et ayant une paroi périphérique intérieure (131) avec un diamètre intérieur (Di) sensiblement égal au diamètre extérieur (De) de ladite tige sensiblement cylindrique (117), lesdits moyens de connexion étant du type écrou et vis (125, 126) et étant associés à ladite paroi périphérique intérieure (131) de ladite saillie (130) et à la surface extérieure (132) de ladite tige (117) au niveau de ladite extrémité distale de connexion (120).

5. Outil selon la revendication 4, caractérisé en ce ledit élément tubulaire mâle (23) est fileté extérieurement, ledit trou central (14) dudit élément en forme de bol (11) ayant une paroi périphérique avec contre-filetage (30) pour permettre l'ancrage par vissage dudit élément mâle (23) même en l'absence dudit élément tubulaire femelle (22).

6. Outil selon la revendication 5, **caractérisé en ce que** ladite tige (17, 117) a un canal transversal auxiliaire en communication fluidique avec ledit passage axial (21, 122) et adapté pour permettre l'évacuation d'air lors de la déformation dudit élément en forme de bol (11, 111).

7. Outil selon la revendication 6, **caractérisé en ce que** ladite tige (17, 117) a des moyens de fin de couse réglables adaptés pour définir une butée réglable pour l'aiguille (2).

8. Outil selon la revendication 7, **caractérisé en ce qu'**il comprend une pluralité de tiges de guidage (17, 117) adaptées pour être couplées de manière amovible et sélective audit élément en forme de bol (101, 111) au niveau dudit trou central (14, 114).

9. Outil selon la revendication 8, **caractérisé en ce que** lesdites tiges (17) de ladite pluralité comprennent des éléments tubulaires mâles (23) et des éléments tubulaires femelles (22) respectifs pouvant être couplés mutuellement, lesdits éléments mâles (23), respectivement femelles (22), étant différenciés les uns des autres par leurs longueurs et/ou leur diamètre intérieur.

10. Outil selon la revendication 9, **caractérisé en ce que** lesdites tiges (17) de ladite pluralité comprennent des paires respectives d'éléments tubulaires mâles (23) et d'éléments tubulaires femelles (22) pouvant être couplés mutuellement, les éléments mâles (23) et/ou les éléments femelles (22) desdites paires étant différenciées entre elles par le diamètre extérieur maximale des brides de serrage correspondantes (27, 28) pour régler le degré de déformabilité dudit élément en forme de bol (11).

11. Ensemble pour l'induction du pneumopéritoine, comprenant :
- une aiguille (2) ayant un corps allongé sensiblement tubulaire (3) avec une extrémité proximale de préhension (4) adaptée pour être manipulée par un opérateur et une extrémité distale effilée opposée (8) adaptée pour pénétrer dans la cavité abdominale d'un patient à travers la paroi abdominale ;
- un outil (10, 110) adapté pour être placé en contact avec la peau d'un patient au niveau de sa paroi abdominale pour la soulever et favoriser l'introduction de ladite aiguille (2) dans la cavité abdominale ;
**caractérisé en ce que** ledit outil (10, 110) est selon une ou plusieurs des revendications précédentes.
